# EUROPÄISCHE PATENTSCHRIFT

(11) **EP 3 087 954 B1**
(45) Veröffentlichungstag und Bekanntmachung des Hinweises auf die Patenterteilung: **30.01.2019**
(21) Anmeldenummer: 15165160.1
(22) Anmeldetag: 27.04.2015
(51) Int. Cl.: A61F 2/28, A61C 8/02

(54) **KIEFERKNOCHENTRANSPLANTATANORDNUNG**
JAW BONE TRANSPLANT ARRANGEMENT
SYSTÈME DE TRANSPLANT D'OS MAXILLAIRE

(43) Veröffentlichungstag der Anmeldung: 02.11.2016
(73) Patentinhaber: Kessler, Peter, 61130 Nidderau (DE)
(72) Erfinder: Kessler, Peter, 61130 Nidderau (DE)
(74) Vertreter: Keil & Schaafhausen Patent- und Rechtsanwälte PartGmbB

(56) Entgegenhaltungen:
- EP-A1- 2 796 109
- WO-A1-96/35391
- WO-A1-2009/004070
- WO-A2-2006/051401
- DE-A1- 10 026 306
- DE-A1-102010 055 433
- DE-A1-102011 011 191
- US-A1- 2009 292 379
- US-A1- 2010 291 508
- US-A1- 2013 090 742

## Beschreibung

Die Erfindung betrifft eine Kieferknochentransplantatanordnung mit einem Knochentransplantat, wobei das Knochentransplantat mindestens eine modellierte erste freie Formfläche aufweist.

Eine derartige Kieferknochentransplantatanordnung ist aus US 2009/0292379 A1 bekannt.

Aus US 2010/0291508 A1 ist eine Kieferknochentransplantatanordnung mit einem Knochentransplantat, das mindestens eine Implantatöffnung aufweist, bekannt.

Eine Folge von Erkrankungen oder Behandlungen eines Kieferknochens kann der Verlust von Kieferknochenmatrix sein. Dieser Verlust kann beispielsweise nach einer Zahnextraktion oder Zystektomie eintreten. Ebenso kann er im Rahmen einer Knochenatrophie als Folge von Zahnverlust oder inflammatorischen Prozessen auftreten. Es stehen dann verschiedene Möglichkeiten zur Verfügung, die verlorene Kieferknochenmatrix zu ersetzen und zu regenerieren.

Eine Möglichkeit ist dabei die Kieferknochenaugmentation mit einem autologen (patienteneigenen) Knochentransplantat. Die Verwendung eines autologen Knochentransplantats ist aufgrund seiner biologischen Aktivität, die durch vitale Zellen und Wachstumsfaktoren bestimmt ist, vorteilhaft. Das autologe Knochentransplantat kann in intraoralen Spenderregionen des Patienten gewonnen werden. Allerdings ist hierbei die Verfügbarkeit ausreichender Mengen begrenzt. Die Entnahme anderweitigen autologen Knochens, beispielsweise vom Beckenkamm des Patienten, erfordert eine zusätzliche Operationsstelle. Es entsteht ein zusätzlicher Knochendefekt mit potentieller Morbidität der Entnahmestelle.

Eine Alternative zur Verwendung eines autologen Knochentransplantats stellt die Applikation von prozessierten allogenen (Knochen von humanen Spendern) Knochentransplantaten dar. Derartige allogene Knochentransplantate können individuell auf den Kieferknochendefekt des Patienten angepasst werden. Hierzu wird ein entsprechendes Knochentransplantat mittels einer Planungssoftware virtuell entworfen und der in Rede stehende Kieferknochendefekt rekonstruiert. Aufgrund des so erstellten Datensatzes kann ein individuelles Knochentransplantat gefräst werden.

Das hergestellte Knochentransplantat kann sodann durch den behandelnden Arzt in den Kieferknochendefekt eingepasst werden. Es wird am Kieferknochen, beispielsweise durch Osteosyntheseschrauben, fixiert. Schließlich wird das Knochentransplantat mit Knochenersatzmaterial und einer Kollagenmembran abgedeckt. Die Kollagenmembran dient hierbei als Resorptionsbarriere.

Die trabekuläre Struktur des Knochentransplantats ermöglicht eine schnelle Aufnahme von Blut und Nährstoffen. Der Inkorporationsprozess des Knochentransplantats weist eine hohe Zuverlässigkeit und Vorhersagbarkeit auf.

Nach Abschluss der Inkorporation des Knochentransplantats kann ein weiterer operativer Schritt erfolgen. In diesem Schritt wird das das Knochentransplantat umgebende Weichgewebe derartig präpariert, dass der behandelnde Arzt Zugang zum Knochentransplantat hat. In das Knochentransplantat können sodann Implantatöffnungen, beispielsweise durch Bohren, eingebracht werden. In die Implantatöffnungen können Implantate inseriert werden. Die Implantate können der Befestigung und Positionierung beispielsweise einer Zahnprothese dienen.

Das Einbringen von Implantatöffnungen in das Knochentransplantat nach bereits erfolgtem Inkorporationsprozess ist jedoch insoweit nachteilhaft, als es eine Schwächung der Primärstabilität von Knochentransplantat und Implantat zur Folge haben kann. Weiterhin wird der Patient auch durch einen erneuten umfangreichen operativen Eingriff belastet. Die Einheilzeit des Knochentransplantats wird somit nicht unerheblich verlängert.

WO 96/35391 A1 zeigt eine Vorrichtung und ein Verfahren zum Verbessern der Form, der Masse und der Festigkeit von alveolaren und intramembranösen Knochen. Hier wird ein Stück Knochen vom bereits existierenden Knochen abgetrennt und mit einer Spanneinrichtung versehen. Der abgetrennte Knochen wächst zunächst mit den verbleibenden Knochen zusammen. Wenn das abgetrennte Knochenteil dann mit Hilfe der Spanneinrichtung unter eine Zugspannung gesetzt wird, bildet sich ein neuer Knochenbereich. Wenn dieser Knochenbereich groß genug ist, kann ein Implantat eingesetzt werden.

DE 10 2010 055 433 A1 zeigt eine Vorrichtung zur Regeneration eines Knochens. Hier wird eine Membran an einer Spanneinrichtung befestigt, die als Schraube ausgebildet ist. Die Membran ist gewölbt, wird mit einer geringen Entfernung zum Knochen angeordnet und mit einer relativ geringen Geschwindigkeit von beispielsweise 1 mm pro Tag vom Knochen weggezogen. Zwischen dem Kieferknochen und der Membran bildet sich dann ein Koagel, in dem sich auch Osteoblasten befinden.

EP 2 796 109 A1 zeigt eine Dentalmembran, die mit Hilfe einer Befestigungseinrichtung gegenüber einem Kieferknochen fixiert wird. Die Membran und der Kieferknochen schließen einen Hohlraum ein, der mit einem Knochentransplantatmaterial gefüllt wird. Die Membran soll dabei dieses Knochentransplantatmaterial an Ort und Stelle halten. In dieses Knochentransplantatmaterial soll dann der Kieferknochen hineinwachsen.

DE 100 26 306 A1 zeigt ein Transplantat, bei dem ein Transplantatkörper mit Schrauben im Kieferknochen befestigt wird. Ein Hohlraum zwischen dem Kieferknochen und den Transplantatkörper ist mit einem Knochenersatzmaterial in Form von Knochenpulver oder Knochenspänen ausgefüllt. Dieses Knochenersatzmaterial soll verhindern, dass die Schleimhaut in den Hohlraum hineinwächst, erlaubt ist aber, dass der Knochen in den Hohlraum hineinwächst.

WO 2009/004070 A1 zeigt ein chirurgisches Implantat, das einen porösen Kern und eine dichte Oberflächenschicht aufweist. Der poröse Kern kann an einen Knochen zur Anlage gebracht werden. Die dichte Oberflächenschicht verhindert, dass weiches Gewebe in den porösen Kern hineinwächst.

US 2013/0090742 A1 beschreibt eine Vorrichtung und ein Verfahren zur Knochenaugmentation. Die Vorrichtung weist eine Superstruktur auf, die porös ist, um ein Hineinwachsen von Knochen zu ermöglichen. Darüber hinaus ist ein Führungsloch vorgesehen, das aufgebohrt werden kann, um ein Implantat aufzunehmen.

Der Erfindung liegt nunmehr die Aufgabe zugrunde, eine Kieferknochentransplantatanordnung bereitzustellen, die eine kurze Einheilzeit aufweist.

Diese Aufgabe wird bei einer Kieferknochentransplantatanordnung der eingangs genannten Art dadurch gelöst, dass das Knochentransplantat mindestens eine Implantatöffnung aufweist und eine Position der Implantatöffnung in dem Knochentransplantat auf eine Position einer Zahnprothese relativ zu dem Knochentransplantat abgestimmt ist, wobei die Implantatöffnung ein Befestigungselement aufweist, das mit dem Knochentransplantat in Eingriff steht, und an dem ein Implantat befestigt, wobei das Befestigungselement als eine Hülse aus einer Metalllegierung oder einem Metall ausgebildet ist, wobei eine axiale Erstreckung des Befestigungselements auf eine axiale Erstreckung der Implantatöffnung abgestimmt ist.

Die erste Freiformfläche ist auf einen Kieferknochendefekt eines Patienten abgestimmt. Eine Freiformfläche ist dabei eine Fläche, die sich als Ganzes nicht durch bekannte geometrische Strukturen darstellen lässt. Derartige bekannte geometrische Strukturen sind beispielsweise Kreise, Ellipsen, Dreiecke, Rechtecke usw. Die Freiformfläche kann beispielsweise durch Verwendung einer entsprechenden Planungssoftware und der dreidimensionalen Visualisierung des Kieferknochendefekts modelliert werden. Das Knochentransplantat kann mit Hilfe der so gewonnenen Daten individuell gefräst werden. Die Implantatöffnung kann zur Insertion von Implantaten Verwendung finden. Eine Position der Implantatöffnung im Knochentransplantat kann ebenso durch die Planungssoftware bestimmt und festgelegt werden. Die Implantatöffnungen können im Fräsprozess in das Knochentransplantat eingebracht werden. Hierdurch ist es möglich, eine Augmentation des Kieferknochens durch das Knochentransplantat bei zeitgleicher Implantation durchzuführen. Im Unterschied zur oben beschriebenen zweiphasigen Augmentation des Kieferknochens kann nunmehr die gesamte Behandlungszeit signifikant verkürzt werden. Ein zweiter umfangreicher operativer Eingriff kann vermieden werden. Die Einheilzeit des Knochentransplantats kann insgesamt verkürzt werden. Die chirurgische Versorgung dreidimensionaler Kieferknochendefekte wird vereinfacht. Die Implantatöffnung weist ein Befestigungselement auf, das mit dem Knochentransplantat in Eingriff steht, und an dem ein Implantat befestigbar ist. Das Befestigungselement wird zeitgleich mit dem Knochentransplantat in den Kieferknochendefekt eingepasst. Es wird erreicht, dass sich während des Inkorporationsprozesses des Knochentransplantats kein Knochengewebe in der Implantatöffnung ausbildet. Die Implantatöffnung bleibt somit auch während des Inkorporationsprozesses intakt und behält ihren vorgesehenen Nenndurchmesser. Das Befestigungselement muss nach erfolgter Inkorporation des Knochentransplantats nicht wieder entfernt werden. Es dient der Befestigung des Implantats. Durch das simultane Einbringen des Knochentransplantats zusammen mit dem Befestigungselement wird eine vergrößerte Primärstabilität des Knochentransplantats und des Implantats erreicht.

Das Befestigungselement ist als eine Hülse aus einer Metalllegierung oder einem Metall ausgebildet, wobei eine axiale Erstreckung des Befestigungselements auf eine axiale Erstreckung der Implantatöffnung abgestimmt ist. Das biologisch inaktive Material des Befestigungselements nimmt nicht am Inkorporationsprozess des Knochentransplantats teil. Weiterhin ist das biologisch inaktive Material sterilisierbar. Vor einer Transplantation des Knochentransplantats mit dem Befestigungselement wird das Befestigungselement sterilisiert. Hierdurch ist gewährleistet, dass das Befestigungselement weitgehend keimfrei ist. Eine Infektion kann vermieden werden. Durch die Abstimmung der axialen Erstreckung des Befestigungselements auf die axiale Erstreckung der Implantatöffnung kann erreicht werden, dass das Befestigungselement vollständig oder zumindest zu einem überwiegenden Teil in der Implantatöffnung aufgenommen wird. Es ist also dann auch bei Vorhandensein des Befestigungselements problemlos möglich, das Knochentransplantat mit einer Kollagenmembran oder umliegendem Weichgewebe abzudecken.

Dabei ist bevorzugt, dass das Knochentransplantat mindestens eine zweite Freiformfläche oder eine Ebene aufweist, die mit der ersten Freiformfläche ein Freiformvolumen einschließt. Das Freiformvolumen bestimmt das Ausmaß der Augmentation des Kieferknochens. Das Freiformvolumen kann dabei durch Anpassung der zweiten Freiformfläche bzw. der Ebene auf die Gegebenheiten des umliegenden Kieferknochens abgestimmt werden. In Bezug auf den Kieferknochen ist somit sowohl eine horizontale als auch eine vertikale Augmentation des Kieferknochens möglich.

Es ist bevorzugt, dass die Implantatöffnung auf einer der ersten Freiformfläche gegenüberliegenden Seite des Knochentransplantats ausgebildet ist. Die erste Freiformfläche ist in ihrer Form an die Form des Kieferknochendefekts angepasst. Die der ersten Freiformfläche gegenüberliegende Seite des Knochentransplantats wird durch die zweite Freiformfläche bzw. die Ebene gebildet. Auf dieser Seite des Knochentransplantats sollen die Implantate inseriert werden, an denen eine Zahnprothese befestigbar ist. Die Umstände, die der behandelnde Arzt am Kieferknochen des Patienten vorfindet, bestimmen, ob diese Seite des Knochentransplantats als zweite Freiformfläche oder als Ebene ausgebildet ist. Die in Rede stehende Seite des Knochentransplantats wird dabei als Ebene ausgebildet sein, wenn beispielsweise die Position der vorgesehenen Zahnprothese dies gestattet. Die Ausbildung einer Ebene an dem Knochentransplantat ist einfacher in der Herstellung als die Ausbildung der zweiten Freiformfläche.

Vorzugsweise ist die Implantatöffnung in dem Freiformvolumen angeordnet, und als Durchgangsöffnung oder Blindöffnung ausgebildet. Die Ausbildung der Implantatöffnung als Durchgangsöffnung ermöglicht die Befestigung des Knochentransplantats am Kieferknochen. Hierzu können beispielsweise Osteosyntheseschrauben in der Implantatöffnung aufgenommen und mit dem Kieferknochen verschraubt werden. Die Osteosyntheseschrauben sind dann in dem Freiformvolumen aufgenommen. Es ist jedoch ebenso möglich, die Implantatöffnung als Blindöffnung auszubilden. Dies ist beispielsweise dann denkbar, wenn eine weniger invasive Art der Befestigung des Knochentransplantats am Kieferknochen möglich ist. Für den Fall, dass die Implantatöffnung als Durchgangsöffnung ausgebildet ist, verbindet die Implantatöffnung die erste Freiformfläche mit der zweiten Freiformfläche bzw. der Ebene. Es ist möglich, die Durchgangsöffnung oder die Blindöffnung beispielsweise durch eine Bohrung in dem vorgefertigten Knochentransplantat anzubringen.

Bei alldem ist bevorzugt, dass das Knochentransplantat mindestens zwei Implantatöffnungen aufweist. In jede der mindestens zwei Implantatöffnungen kann ein Implantat inseriert werden. Es ist somit möglich, die Kieferknochentransplantatanordnung über die Verwendung bei einer Einzelzahnlücke hinaus zu gebrauchen. An jedem der Implantate, die in den Implantatöffnungen aufgenommen werden können, kann eine entsprechende Zahnprothese befestigt werden.

Auch ist bevorzugt, dass das Befestigungselement aus Titan ausgebildet ist. In eine derartige Hülse kann ein Implantat ohne weiteres inseriert werden. Es ist möglich, die verwendeten Hülsen auf die benötigten Implantate abzustimmen. Es können beispielsweise genormte Bohrhülsen verwendet werden. Derartige Bohrhülsen sind vorzugsweise aus Titan ausgebildet. Ein Metall oder eine Metalllegierung ist dabei biologisch inaktiv und einfach sterilisierbar.

Auch ist bevorzugt, dass eine Position der Implantatöffnung in dem Knochentransplantat auf eine Position einer Zahnprothese relativ zu dem Knochentransplantat abgestimmt ist. Die Position der Implantatöffnung im Knochentransplantat wird dabei so gewählt, dass sich die Zahnprothese ohne weiteres in die Umgebung des Knochentransplantats integrieren lässt. Die Planung der Position der Implantatöffnung im Knochentransplantat kann dabei bereits während der Planung des Knochentransplantats und der beabsichtigten Augmentation erfolgen.

Schließlich ist bevorzugt, dass die Zahnprothese an dem Implantat befestigbar ist. Hierdurch wird erreicht, dass nach erfolgtem Inkorporationsprozess des Knochentransplantats und erneuter Entfernung des umliegenden Weichgewebes in einem zweiten Operationsschritt die vorgesehene Zahnprothese ohne weiteres an dem bereits in dem Knochentransplantat befindlichen Implantat befestigt werden kann. Ein größerer invasiver Eingriff kann vermieden werden. Hierdurch kann die Einheilzeit der Kieferknochentransplantatanordnung herabgesetzt werden.

Die Erfindung wird im Folgenden anhand von bevorzugten Ausführungsbeispielen in Verbindung mit den Zeichnungen beschrieben. Hierin zeigen:
- Fig. 1: eine Kieferknochentransplantatanordnung mit einem Knochentransplantat, das eine Implantatöffnung aufweist,
- Fig. 2: eine weitere Darstellung der Kieferknochentransplantatanordnung der Fig. 1,
- Fig. 3: eine zweite Kieferknochentransplantatanordnung mit drei Implantatöffnungen.

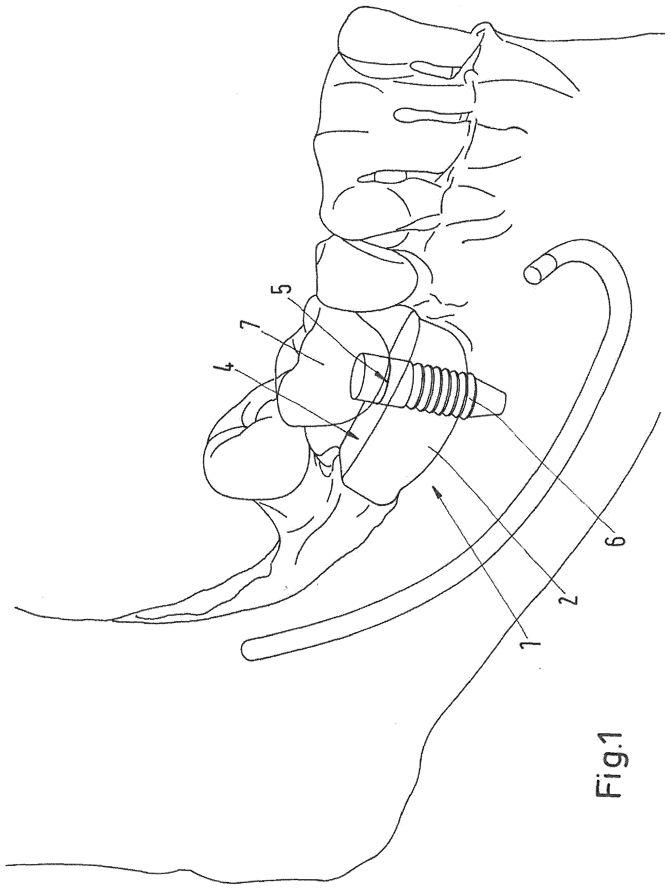

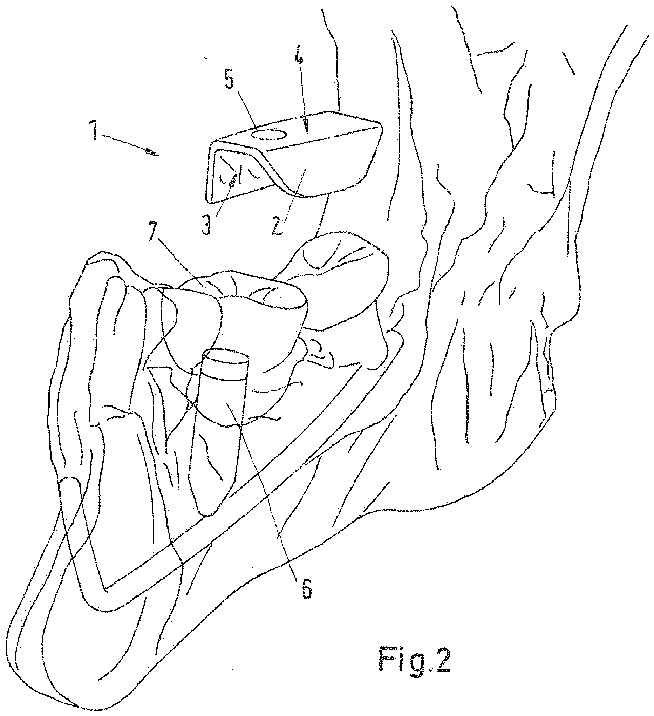

Fig. 1 zeigt eine Kieferknochentransplantatanordnung 1 mit einem Knochentransplantat 2. Die Kieferknochentransplantatanordnung 1 weist ein Freiformvolumen auf, das von einer ersten Freiformfläche 3 und einer Ebene 4 eingeschlossen wird. In der Ebene 4 ist eine erste Implantatöffnung 5 angeordnet. In der ersten Implantatöffnung 5 ist ein Implantat 6 aufgenommen. Hierzu ist die Implantatöffnung 5 als Durchgangsöffnung ausgebildet. Bei dem Implantat 6 handelt es sich in dem Ausführungsbeispiel der Fig. 1 und 2 um eine Osteosyntheseschraube. An dem Implantat 6 ist eine Zahnprothese 7 befestigt.

Insbesondere in Fig. 2 ist zu erkennen, dass die erste Freiformfläche 3 auf einen Kieferknochendefekt abgestimmt ist. Es lässt sich somit ein genauer Sitz des Knochentransplantats 2 am Kieferknochen ermöglichen. Die der ersten Freiformfläche 3 gegenüberliegende Seite des Knochentransplantats 2 ist als Ebene 4 ausgebildet, was durch die Struktur des Kieferknochendefekts möglich und bedingt ist. Die erste Freiformfläche 3 und die Ebene 4 schließen das Freiformvolumen ein. Dieses Freiformvolumen legt die Augmentation, die durch das Knochentransplantat 2 bereitgestellt wird, fest.

Die Herstellung des Knochentransplantats 2 kann in folgenden Schritten erfolgen. In einem ersten Schritt wird ein virtuelles dreidimensionales Planungsmodell des Kieferknochendefekts erstellt. Mit einer Planungssoftware wird sodann die Struktur des Knochentransplantats 2 bestimmt. Das Knochentransplantat 2 kann anschließend in einem Fräsprozess aus einem Knochenblock hergestellt werden. Die Struktur des Knochentransplantats 2 ist dabei durch die erste Freiformfläche 3, eine zweite Freiformfläche bzw. die Ebene 4 sowie die Implantatöffnung 5 bestimmt.

Bevor das Knochentransplantat 2 in den Kieferknochen des Patienten transplantiert wird, kann ein Befestigungselement (nicht dargestellt) in der Implantatöffnung 5 angeordnet und aufgenommen werden. Hierzu ist die Implantatöffnung 5 als Durchgangsöffnung oder Blindöffnung ausgebildet.

Das Befestigungselement steht mit dem Knochentransplantat 2 in Eingriff. An diesem ist das Implantat 6 befestigbar. Das Befestigungselement ist dabei vorzugsweise aus einem biologisch inaktiven Material ausgebildet. Hierdurch kann zum einen vermieden werden, dass während des Inkorporationsprozesses des Knochentransplantats 2 ein Verschluss der Implantatöffnung durch Knochengewebe entsteht. Die Implantatöffnung 5 kann auf ihrem vorgesehenen Durchmesser gehalten werden. Zum anderen wird das Befestigungselement aus einem sterilen bzw. sterilisierbaren Material ausgebildet sein. Hierdurch lassen sich Infektionen des Knochentransplantats 2 vermeiden.

Um eine Abdeckung des Knochentransplantats 2 mit einer Kollagenmembran bzw. umliegendem Weichgewebe zu ermöglichen, ist eine axiale Erstreckung des Befestigungselements auf eine axiale Erstreckung der Implantatöffnung 5 abgestimmt. Das Befestigungselement kann also beispielsweise derartig ausgebildet sein, dass es vollständig oder zumindest zu einem großen Teil in der Implantatöffnung 5 aufgenommen werden kann.

Es ist dabei besonders bevorzugt, das Befestigungselement als eine Hülse auszubilden. Diese Hülse kann genormte Abmessungen aufweisen. Sie kann beispielsweise als Bohrhülse ausgebildet sein. Die Bohrhülse lässt sich dabei aus einem Metall oder einer Metalllegierung ausbilden. Als Metall kommt beispielsweise Titan infrage. Eine Hülse aus einem Metall oder einer Metalllegierung ist dabei einfach sterilisierbar.

Weiterhin ist eine Position der Implantatöffnung 5 in dem Knochentransplantat 2 auf eine Position der Zahnprothese 7 relativ zu dem Knochentransplantat 2 abgestimmt. Die Zahnprothese 7 kann somit passgenau in die umliegenden Zähne und die umliegende Kieferknochenstruktur integriert werden. Die Zahnprothese 7 ist an dem Implantat 6 befestigbar.

Die Augmentation des Kieferknochens ist nun gleichzeitig mit der Implantation durchführbar. Hierdurch kann die Behandlungszeit des Patienten erheblich verkürzt werden. Ein erneuter Eingriff zum Setzen der Implantatöffnung 5 und zum Einbringen des Implantats 6 nach dem erfolgten Inkorporationsprozess des Knochentransplantats 2 kann entfallen. Durch das simultane Setzen des Knochentransplantats 2 mit dem Implantat 6 wird eine vergrößerte Primärstabilität von Knochentransplantat 2 und Implantat 6 erreicht. Es lässt sich eine vereinfachte chirurgische Versorgung dreidimensionaler Kieferknochendefekte erreichen. Die Zuverlässigkeit der erfindungsgemäßen Kieferknochentransplantatanordnung 1 im klinischen Alltag wird erhöht. Die Einheilzeiten der Kieferknochentransplantatanordnung 1 können verkürzt werden.

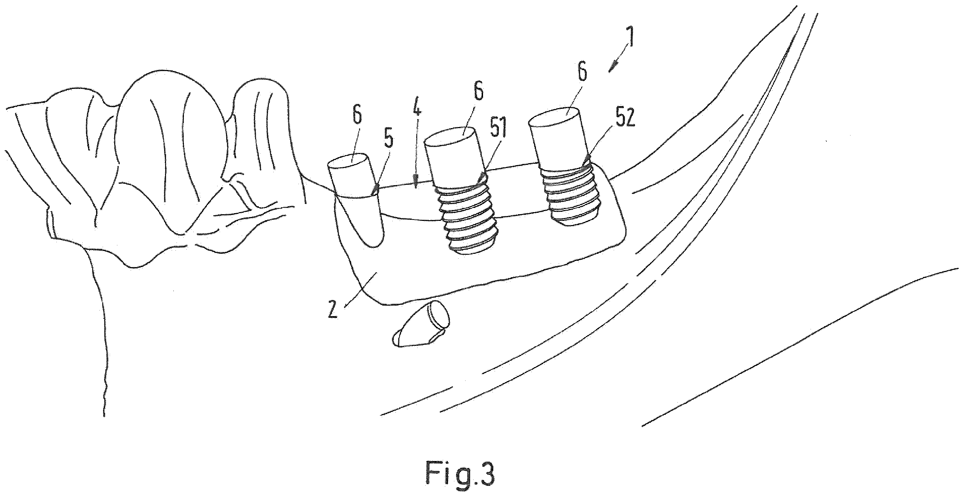

Fig. 3 zeigt eine Kieferknochentransplantatanordnung 1 mit einer ersten Implantatöffnung 5, einer zweiten Implantatöffnung 51 und einer dritten Implantatöffnung 52. Im Übrigen sind identische technische Merkmale mit identischen Bezugszeichen versehen.

Die Kieferknochentransplantatanordnung 1 lässt sich über eine einzelne Zahnlücke hinaus auch für die horizontale und vertikale Augmentation des Kieferknochens nutzen, die mehr als eine Zahnprothese 7 umfassen soll.

## Patentansprüche

1. Kieferknochentransplantatanordnung (1) mit einem Knochentransplantat (2), wobei das Knochentransplantat (2) mindestens eine modellierte erste Freiformfläche (3) aufweist, **dadurch gekennzeichnet, dass** das Knochentransplantat mindestens eine Implantatöffnung (5) aufweist und eine Position der Implantatöffnung (5) in dem Knochentransplantat (2) auf eine Position einer Zahnprothese (7) relativ zu dem Knochentransplantat (2) abgestimmt ist, wobei die Implantatöffnung (5) ein Befestigungselement aufweist, das mit dem Knochentransplantat (2) in Eingriff steht, und an dem ein Implantat (6) befestigbar ist, wobei das Befestigungselement als eine Hülse aus einer Metalllegierung oder einem Metall ausgebildet ist, wobei eine axiale Erstreckung des Befestigungselements auf eine axiale Erstreckung der Implantatöffnung (5) abgestimmt ist.

2. Kieferknochentransplantatanordnung nach Anspruch 1, **dadurch gekennzeichnet, dass** das Knochentransplantat (2) mindestens eine zweite Freiformfläche oder eine Ebene (4) aufweist, die mit der ersten Freiformfläche (3) ein Freiformvolumen einschließt.

3. Kieferknochentransplantatanordnung nach einem der Ansprüche 1 oder 2, **dadurch gekennzeichnet, dass** die Implantatöffnung (5) auf einer der ersten Freiformfläche (3) gegenüberliegenden Seite des Knochentransplantats (2) ausgebildet ist.

4. Kieferknochentransplantatanordnung nach Anspruch 2 oder 3, **dadurch gekennzeichnet, dass** die Implantatöffnung (5) in dem Freiformvolumen angeordnet ist, und als Durchgangsöffnung oder Blindöffnung ausgebildet ist.

5. Kieferknochentransplantatanordnung nach einem der Ansprüche 1 bis 4, **dadurch gekennzeichnet, dass** das Knochentransplantat (2) mindestens zwei Implantatöffnungen (5, 51, 52) aufweist.

6. Kieferknochentransplantatanordnung nach einem der Ansprüche 1 bis 5, **dadurch gekennzeichnet, dass** das Befestigungselement aus Titan ausgebildet ist.

7. Kieferknochentransplantatanordnung nach Anspruch 1, **dadurch gekennzeichnet, dass** die Zahnprothese (7) an dem Implantat (6) befestigbar ist.

## Claims

1. Jawbone transplant arrangement (1) having a bone transplant (2), wherein the bone transplant (2) has at least one modelled first free-form surface (3), **characterised in that** the bone transplant has at least one implant opening (5) and a position of the implant opening (5) in the bone transplant (2) is aligned with a position of a dental plate (7) relatively to the bone transplant (2), wherein the implant opening (5) has a fastening element which engages with the bone transplant (2), and at which an implant (6) can be fastened, wherein the fastening element is formed as a sheath from a metal alloy or a metal, wherein an axial extension of the fastening element is aligned with an axial extension of the implant opening (5).

2. Jawbone transplant arrangement according to claim 1, **characterised in that** the bone transplant (2) has at least one second free-form surface or a plane (4) which encloses with the first free-form surface (3) a free-form volume.

3. Jawbone transplant arrangement according to any of claims 1 or 2, **characterised in that** the implant opening (5) is formed on a side of the bone transplant (2) opposite the first free-form surface (3).

4. Jawbone transplant arrangement according to claim 2 or 3, **characterised in that** the implant opening (5) is disposed in the free-form volume and is in the form of through opening or blind opening.

5. Jawbone transplant arrangement according to any of claims 1 to 4, **characterised in that** the bone transplant (2) has at least two implant openings (5, 51, 52).

6. Jawbone transplant arrangement according to any of claims 1 to 5, **characterised in that** the fastening element is formed from titanium.

7. Jawbone transplant arrangement according to claim 1, **characterised in that** the dental plate (7) can be fastened to the implant (6).

## Revendications

1. Système de transplant d'os maxillaire (1) avec un transplant d'os (2), dans lequel le transplant d'os (2) présente au moins une première surface de forme libre modelée (3), **caractérisé en ce que** le transplant d'os présente au moins une ouverture d'implant (5) et une position de l'ouverture d'implant (5) dans le transplant d'os (2) est adaptée à une position d'une prothèse dentaire (7) par rapport au transplant d'os (2), dans lequel l'ouverture d'implant (5) présente un élément de fixation, qui est en prise avec le transplant d'os (2), et sur lequel un implant (6) peut être fixé, dans lequel l'élément de fixation est réalisé en tant que douille en un alliage métallique ou en un métal, dans lequel une extension axiale de l'élément de fixation est adaptée à une extension axiale de l'ouverture d'implant (5).

2. Système de transplant d'os maxillaire selon la revendication 1, **caractérisé en ce que** le transplant d'os (2) présente au moins une deuxième surface de forme libre ou un plan (4), qui inclut un volume de forme libre avec la première surface de forme libre (3).

3. Système de transplant d'os maxillaire selon l'une quelconque des revendications 1 ou 2, **caractérisé en ce que** l'ouverture d'implant (5) est réalisée sur un côté du transplant d'os (2) opposé à la première surface de forme libre (3).

4. Système de transplant d'os maxillaire selon la revendication 2 ou 3, **caractérisé en ce que** l'ouverture d'implant (5) est agencée dans le volume de forme libre, et est réalisée en tant qu'ouverture traversante ou ouverture aveugle.

5. Système de transplant d'os maxillaire selon l'une quelconque des revendications 1 à 4, **caractérisé en ce que** le transplant d'os (2) présente au moins deux ouvertures d'implant (5, 51, 52).

6. Système de transplant d'os maxillaire selon l'une quelconque des revendications 1 à 5, **caractérisé en ce que** l'élément de fixation est réalisé en titane.

7. Système de transplant d'os maxillaire selon la revendication 1, **caractérisé en ce que** la prothèse dentaire (7) peut être fixée sur l'implant (6).
